# EUROPEAN PATENT APPLICATION

(11) **EP 4 574 975 A1**
(43) Date of publication of application: **25.06.2025**
(21) Application number: 23218469.7
(22) Date of filing: 20.12.2023
(51) Int. Cl.: C12N 15/11, C12N 15/90, C12N 9/22

(54) **COMPOSITIONS AND METHODS FOR DELIVERING TRANSGENES**

(71) Applicant: Bayer AG, 51373 Leverkusen (DE)
(72) Inventor: The designation of the inventor has not yet been filed
(74) Representative: BIP Patents

(57) **Abstract**

Disclosed herein are compositions and methods that allow a high efficiency integration of genes of interest into a safe harbour locus in the genome. Such compositions and methods comprise small type V nucleases, guide RNAs, preferably targeting the Albumin locus.

## Description

### 1. Background

The disclosures provide compositions, methods, and systems for targeted delivery of nucleic acids to a target cell such as, e.g., human cell. Some embodiments of the invention relate to compositions, methods, and systems for expressing a transgene in a cell by genome editing.

### BACKGROUND

Recent advances in genome sequencing techniques and analytical methods have significantly accelerated the ability to catalog and map genetic factors associated with a diverse range of biological functions and diseases. Precise genome targeting technologies are needed to enable systematic reverse engineering of causal genetic variations by allowing selective perturbation of individual genetic elements, as well as to advance synthetic biology, biotechnological, and medical applications.

Recently, gene editing using designed site-specific nucleases emerged as a technology for both basic biomedical research and therapeutic development. In the recent years, various platforms based on four major types of endonucleases have been developed for gene editing, namely the meganucleases and their derivatives, the zinc finger nucleases (ZFNs), the transcription activator-like effector nucleases (TALENs), and the clustered regularly interspaced short palindromic repeat (CRISPR) associated endonuclease 9 (Cas9). Each nuclease type is capable of inducing a DNA double-stranded break (DSB) at specific DNA loci, thus triggering two DNA repair pathways. The non-homologous end joining (NHEJ) pathway generates random insertion/deletion (indel) mutations at the DSB, whereas the homology-directed repair (HDR) pathway repairs the DSB with the genetic information carried on a donor template. Therefore, these gene editing platforms can manipulate genes at specific genomic loci in multiple ways, such as disrupting gene function, repairing a mutant gene to normal, and inserting DNA material.

However, presently available genome editing techniques can suffer from a number of disadvantages, for example, inaccuracy, unacceptable levels of off-target editing, etc.

Accordingly, there remains a need for new genome gene editing platforms that are capable of manipulating genes at specific genomic loci in multiple ways, such as disrupting gene function, repairing a mutant gene to normal, and/or inserting heterologous DNA material at specific loci, such as a safe harbor locus within the genome of a target cell.

Cell and gene therapy offers unprecedented opportunities to treat the untreatable. Especially in pedriatric patients standard gene therapy by augmentation of episomal AAV vectors is hampered by organ growth. This leads to dilution of the vectors and consequently lowers therapeutic effects. To mitigate this downside stable gene modification is desired for gene therapeutic applications.

Albumin is an attractive target for gene modifying gene therapy, since it is expressed highly liver specific and offers an intronic safe harbour platform for the stable integration and expression of therapeutic transgenes. RNA-programmable nucleases offer a highly dynamic and versatile tool for targeted modification of genomic sequences. Those nucleases enable the framework for integration of templates of choice by cutting open the target site, which is a prerequisite for integration.

Until now the high opportunity target albumin was merely approachable with Cas9 from *B. pyogenes.* However, there is a need for the utilization of this principle with significantly smaller CRISPR Cas effectors, that in further might also have other additional beneficial properties over the systems of the prior art, as e.g. higher nuclease activity, higher selectivity or less complex PAM requirements. The recently developed Type V B-Gen-1 and 2 CRISPR Cas nucleases (WO2022258753) provide small sized effectors with low complex PAM requirements.

We herewith provide methods and compositions that allow the use of small sized CRISPR Cas nucleases (B-Gen.1 (SEQ ID NO:185 or SEQ NO: 186) and polypetides of at least 95% sequence identity to SEQ ID NOs: 185 and 186, for the integration of genes of interest (GOIs) into a safe harbour locus, preferably into the albumin locus.

Another aspect of the compositions and methods according to the invention is that surprisingly high activities of the GOIs can be obtained.

WO2020/082042 discloses methods and reagents based on *S.pyogenes* CRISPR Cas9 nucleases to integrate transgenes into an albumin locus. WO2020/081843 also discloses such systems based on various Cas9 (Type II) CRISPR nucleases.

WO2022258753 discloses the type V CRISPR Cas nucleases utilized in this invention, with the proviso that the nuclease named B-Gen.1 in this application (SEQ ID NO: 185) is named B-Gen.2 (SEQ ID NO: 2) in WO2022258753.

### 2. Summary of the invention

Provided herein, inter alia, are compositions, methods, and systems for targeted delivery of nucleic acids, including DNA and RNA, to a target cell, e.g., human cell. Also provided are compositions, methods, and systems for expressing a transgene in a cell by integration of the transgene into the genome of the cell in a targeted manner by genomic editing. Certain aspects and embodiments of the disclosure concern compositions, methods, and systems for knocking in a gene-of-interest (GOI) into a specific safe habor location in the genome, in particular to a genomic location within or near an endogenous albumin locus. Further provided are compositions, methods, and systems for treating a subject having or suspected of having a disorder or health condition employing ex vivo and/or in vivo genome editing.

In one aspect, provided herein is a guide RNA (gRNA) sequence having a sequence that is complementary to a genomic sequence within or near an endogenous albumin locus.

In some embodiments, the gRNA has a sequence selected from those of SEQ ID NOs: 62 to 122, and variants thereof having at least 85%, preferably 90%, more preferably 95%, even more preferably 98% identity to any of those sequences.

In another aspect, provided herein is a composition having any of the above-mentioned gRNAs.

In one aspect, provided herein is a system including a guide RNA (gRNA), comprising a spacer, as disclosed herein or a nucleic acid encoding the gRNA. In some embodiments, the gRNA of the system has a sequence selected from SEQ ID NOs: 62 to 122 and variants thereof having at least 85%, preferably 90%, more preferably 95%, even more preferably 98% identity to any of those sequences. In some preferred embodiments, the gRNA comprises a guide RNA (gRNA) sequence listed in Table 1 for preferred, more preferred, particularly preferred, more particularly preferred, or most preferred gRNA sequences.

**Table 1:**

| **SEQ ID NO:** | ***preferred*** | ***more preferred*** | ***particularly preferred*** | ***more particularly preferred*** | ***most preferred*** |
|---|---|---|---|---|---|
| | 62 | 64 | 65 | 65 | 65 |
| | 63 | 65 | 66 | 66 | 103 |
| | 64 | 66 | 67 | 67 | |
| | 65 | 67 | 70 | 70 | |
| | 66 | 70 | 73 | 76 | |
| | 67 | 72 | 74 | 81 | |
| | 68 | 73 | 75 | 103 | |
| | 69 | 74 | 76 | 113 | |
| | 70 | 75 | 81 | 120 | |
| | 72 | 76 | 87 | | |
| | 73 | 77 | 93 | | |
| | 74 | 81 | 100 | | |
| | 75 | 82 | 103 | | |
| | 76 | 83 | 113 | | |
| | 77 | 86 | 115 | | |
| | 78 | 87 | 116 | | |
| | 79 | 93 | 119 | | |
| | 80 | 100 | 120 | | |
| | 81 | 103 | | | |
| | 82 | 105 | | | |
| | 83 | 107 | | | |
| | 84 | 112 | | | |
| | 85 | 113 | | | |
| | 86 | 115 | | | |
| | 87 | 116 | | | |
| | 88 | 117 | | | |
| | 89 | 119 | | | |
| | 90 | 120 | | | |
| | 91 | 121 | | | |
| | 92 | | | | |
| | 93 | | | | |
| | 94 | | | | |
| | 96 | | | | |
| | 100 | | | | |
| | 101 | | | | |
| | 102 | | | | |
| | 103 | | | | |
| | 104 | | | | |
| | 105 | | | | |
| | 106 | | | | |
| | 107 | | | | |
| | 108 | | | | |
| | 109 | | | | |
| | 110 | | | | |
| | 112 | | | | |
| | 113 | | | | |
| | 115 | | | | |
| | 116 | | | | |
| | 117 | | | | |
| | 119 | | | | |
| | 120 | | | | |
| | 121 | | | | |

In some embodiments, the system further has one or more of the following: a deoxyribonucleic acid (DNA) endonuclease or a nucleic acid encoding the DNA endonuclease; and a donor template having a nucleic acid sequence encoding a gene-of-interest (GOI) or functional derivative thereof. In some embodiments, the gRNA comprises a spacer sequence from any one of SEQ ID NOs: 1 to 61. In some preferred embodiments, the gRNA comprises a spacer sequence listed in Table 2 for preferred, more preferred, particularly preferred, more particularly preferred, or most preferred spacer sequences.

**Table 2:**

| **SEQ ID NO:** | ***preferred*** | ***more preferred*** | ***particularly preferred*** | ***more particularly preferred*** | ***most preferred*** |
|---|---|---|---|---|---|
| | 1 | 3 | 4 | 4 | 4 |
| | 2 | 4 | 5 | 5 | 42 |
| | 3 | 5 | 6 | 6 | |
| | 4 | 6 | 9 | 9 | |
| | 5 | 9 | 12 | 15 | |
| | 6 | 11 | 13 | 20 | |
| | 7 | 12 | 14 | 42 | |
| | 8 | 13 | 15 | 52 | |
| | 9 | 14 | 20 | 59 | |
| | 11 | 15 | 26 | | |
| | 12 | 16 | 32 | | |
| | 13 | 20 | 39 | | |
| | 14 | 21 | 42 | | |
| | 15 | 22 | 52 | | |
| | 16 | 25 | 54 | | |
| | 17 | 26 | 55 | | |
| | 18 | 32 | 58 | | |
| | 19 | 39 | 59 | | |
| | 20 | 42 | | | |
| | 21 | 44 | | | |
| | 22 | 46 | | | |
| | 23 | 51 | | | |
| | 24 | 52 | | | |
| | 25 | 54 | | | |
| | 26 | 55 | | | |
| | 27 | 56 | | | |
| | 28 | 58 | | | |
| | 29 | 59 | | | |
| | 30 | 60 | | | |
| | 31 | | | | |
| | 32 | | | | |
| | 33 | | | | |
| | 35 | | | | |
| | 39 | | | | |
| | 40 | | | | |
| | 41 | | | | |
| | 42 | | | | |
| | 43 | | | | |
| | 44 | | | | |
| | 45 | | | | |
| | 46 | | | | |
| | 47 | | | | |
| | 48 | | | | |
| | 49 | | | | |
| | 51 | | | | |
| | 52 | | | | |
| | 54 | | | | |
| | 55 | | | | |
| | 56 | | | | |
| | 58 | | | | |
| | 59 | | | | |
| | 60 | | | | |

In some embodiments, the DNA endonuclease recognizes a protospacer adjacent motif (PAM) having the sequence "DTTN" with "D" representing "A" or "T" or "G"; preferably NGG or NNGG, wherein N is any nucleotide, or a functional derivative thereof, or a functional derivative thereof.

In some embodiments, the deoxyribonucleic acid (DNA) endonuclease is a type V nuclease. In some embodiments, the deoxyribonucleic acid (DNA) endonuclease is selected from SEQ ID NO: 185 or SEQ ID NO: 186, or any sequence thereof which is 95%, preferably, 98%, more preferably 99% identical to these sequences.

In some embodiments, the nucleic acid encoding the DNA endonuclease is codon optimized for expression in a host cell.

In some embodiments, the nucleic acid sequence encoding the gene-of-interest (GOI) is codon optimized for expression in a host cell. In some embodiments, the GOI encodes a polypeptide selected from the group consisting of a therapeutic polypeptide and a prophylactic polypeptide. In some embodiments, the GOI encodes a protein selected from the group consisting of Factor VIII (FVIII) protein, Factor IX protein, alpha- 1 -antitrypsin, Factor XIII (FXIII) protein, Factor VII (FVII) protein, Factor X (FX) protein, Protein C, serine protease inhibitor GI (Serpin GI), or a functional derivative of any thereof. In some embodiments, the GOI encodes a FVIII protein or a functional derivative thereof. In some embodiments, the GOI encodes a FIX protein or a functional derivative thereof. In some embodiments, the GOI encodes a serpin GI protein or a functional derivative thereof.

In some embodiments, the nucleic acid encoding the DNA endonuclease is a deoxyribonucleic acid (DNA) sequence.

In some embodiments, the nucleic acid encoding the DNA endonuclease is a ribonucleic acid (RNA) sequence.

In some embodiments, the RNA sequence encoding the DNA endonuclease is linked to the gRNA via a covalent bond.

In some embodiments, the composition further has a liposome or lipid nanoparticle.

In some embodiments, the donor template is encoded in a viral vector.

In some embodiments, the donor template is encoded in an Adeno Associated Virus (AAV) vector.

In some embodiments, the DNA endonuclease is formulated in a liposome or lipid nanoparticle.

In some embodiments, the liposome or lipid nanoparticle also has the gRNA.

In some embodiments the components of the composition (DNA endonuclease, guide RNA, and donor template) are encoded by a single viral vector.

In some embodiments the components of the composition (DNA endonuclease, guide RNA, and donor template) are encoded by separate viral vectors, including embodiments wherein one or two components are encoded by a single viral vector.

In some embodiments, the DNA endonuclease is precomplexed with the gRNA, forming a ribonucleoprotein (RNP) complex.

In another aspect, provided herein is a kit having any of the composition described above and further having instructions for use.

In another aspect, provided herein is a method of editing a genome in a cell. The method includes providing the following to the cell: (a) any of the gRNA described herein or nucleic acid encoding the gRNA; (b) a deoxyribonucleic acid (DNA) endonuclease or a nucleic acid encoding the DNA endonuclease; and (c) a donor template having a nucleic acid sequence encoding a gene-of-interest (GOI) or functional derivative. In some embodiments, the gRNA comprises a spacer sequence from any one of SEQ ID NOs: listed in Table 1.

In some embodiments, the gRNA has a sequence selected from those listed in Table 1 and variants thereof having at least 85% homology to any of those listed in Table 1.

In some embodiments, the nucleic acid sequence encoding the gene-of-interest (GOI) is codon optimized. In some embodiments, the GOI encodes a polypeptide selected from the group consisting of a therapeutic polypeptide and a prophylactic polypeptide. In some embodiments, the GOI encodes a protein selected from the group consisting of FVIII protein, FIX protein, alpha- 1 -antitrypsin, FXIII protein, FVII protein, FX protein, Protein C, Serpin GI, or a functional derivative of any thereof.

In some embodiments, the nucleic acid sequence encoding the gene-of-interest (GOI) is inserted into a genomic sequence of the cell.

In some embodiments, the insertion is at, within, or near the albumin gene or albumin gene regulatory elements in the genome of the cell.

In some embodiments, the insertion is in the first intron of the albumin gene.

In some embodiments, the insertion is at least 37 bp downstream of the end of the first exon of the human albumin gene in the genome and at least 330 bp upstream of the start of the second exon of the human albumin gene in the genome.

In some embodiments, the nucleic acid sequence encoding the gene-of-interest is expressed under the control of the endogenous albumin promoter.

In some embodiments, the cell is a hepatocyte.

In another aspect, provided herein is a genetically modified cell in which the genome of the cell is edited by any of the method described above.

In some embodiments, the nucleic acid sequence encoding the gene-of-interest is inserted into a genomic sequence of the cell.

In some embodiments, the insertion is at, within, or near the albumin gene or albumin gene regulatory elements in the genome of the cell.

In some embodiments, the insertion is in the first intron of the albumin gene.

In some embodiments, the nucleic acid sequence encoding the gene-of-interest is expressed under the control of the endogenous albumin promoter.

In some embodiments, the nucleic acid sequence encoding the gene-of-interest is codon optimized. In some embodiments, the cell is a hepatocyte.

In another aspect, provided herein is a method of treating a disorder or health condition in a subject. The method includes administering any of the above-mentioned genetically modified cell to the subject.

In some embodiments, the genetically modified cell is autologous.

In some embodiments, the method further has obtaining a biological sample from the subject wherein the biological sample has a hepatocyte cell and editing the genome of the hepatocyte cell by inserting a nucleic acid sequence encoding the gene-of-interest thereof into a genomic sequence of the cell, thereby producing the genetically modified cell.

In another aspect, provided herein is a method of treating a disorder or health condition in a subject. The method has obtaining a biological sample from the subject wherein the biological sample has a hepatocyte cell, providing the following to the hepatocyte cell: (a) any of the gRNA described above or nucleic acid encoding the gRNA; (b) a deoxyribonucleic acid (DNA) endonuclease or a nucleic acid encoding the DNA endonuclease; and (c) a donor template having a nucleic acid sequence encoding a gene-of-interest (GO I) or functional derivative, thereby producing a genetically modified cell, and administering the genetically modified cell to the subject.

In some embodiments, the subject is a patient having or suspected of having a disorder or health condition selected from the group consisting of FVIII deficiency (hemophilia A), FIX deficiency (hemophilia B), Hunters Syndrome (MPS II), Hurlers Syndrome (MPS1H), alpha-I- antitrypsin deficiency, FXIII deficiency, FVII deficiency, FX deficiency, Protein C deficiency, and hereditary angioedema (HAE). In some embodiments, the subject is a patient having or suspected of having hemophilia A. In some embodiments, the subject is a patient having or suspected of having hemophilia B. In some embodiments, the subject is a patient having or suspected of having HAE.

In some embodiments, the subject is diagnosed with a risk of a disorder or health condition selected from the group consisting of hemophilia A, hemophilia B, MPS II, MPS1H, alpha- 1 -antitrypsin deficiency, FXIII deficiency, FVII deficiency, FX deficiency, Protein C deficiency, and HAE. In some embodiments, the subject is diagnosed with a risk of hemophilia A. In some embodiments, the subject is diagnosed with a risk of hemophilia B. In some embodiments, the subject is diagnosed with a risk of HAE.

Each of the aspects and embodiments described herein are capable of being used together, unless excluded either explicitly or clearly from the context of the embodiment or aspect.

The foregoing summary is illustrative only and is not intended to be in any way limiting. In addition to the illustrative embodiments and features described herein, further aspects, embodiments, objects and features of the disclosure will become fully apparent from the drawings and the detailed description and the claims.

**3. Table of Sequences**

| **SEQ ID NO:** | **Identifier** | **Sequence** |
|---|---|---|
| 1 | insert of pBLR2542 | TTCTATTGTTCAACTTTTATTCT |
| 2 | insert of pBLR2543 | TTCAACTTTTATTCTATTTTCCC |
| 3 | insert of pBLR2545 | TATTTTCCCAGTAAAATAAAGTT |
| 4 | insert of pBLR2546 | TCCCAGTAAAATAAAGTTTTAGT |
| 5 | insert of pBLR2544 | TACTGGGAAAATAGAATAAAAGT |
| 6 | insert of pBLR2547 | TTTAAAGATGCAGAGTTTACTAA |
| 7 | insert of pBLR2549 | TTTTGGCATTTATTTCTAAAATG |
| 8 | insert of pBLR2550 | TGGCATTTATTTCTAAAATGGCA |
| 9 | insert of pBLR2551 | ATTTCTAAAATGGCATAGTATTT |
| 10 | insert of pBLR2552 | CTAAAATGGCATAGTATTTTGTA |
| 11 | insert of pBLR2548 | TAGAAATAAATGCCAAAATAATT |
| 12 | insert of pBLR2553 | TGTATTTGTGAAGTCTTACAAGG |
| 13 | insert of pBLR2554 | GTGAAGTCTTACAAGGTTATCTT |
| 14 | insert of pBLR2557 | ATAAAATTCAAACATCCTAGGTA |
| 15 | insert of pBLR2555 | ATAAGATAACCTTGTAAGACTTC |
| 16 | insert of pBLR2559 | TGACCTTTTTTTTTTTTTACCTA |
| 17 | insert of pBLR2561 | TTTAGTGACTGTAATTTTCTTTT |
| 18 | insert of pBLR2560 | CAGTCACTAAACAATTCTGACCT |
| 19 | insert of pBLR2562 | TCTTTTGCGCACTAAGGAAAGTG |
| 20 | insert of pBLR2563 | TGTGAAGTTTCAGTCACTCTAAG |
| 21 | insert of pBLR2565 | AATTCATAACTATCCCAAAGACC |
| 22 | insert of pBLR2564 | AATCTTCAACCCTATTCTGTGAA |
| 23 | insert of pBLR2537 | ATAACTATCCCAAAGACCTATCC |
| 24 | insert of pBLR2538 | CACTATGCTTTATTTAAAAACCA |
| 25 | insert of pBLR2539 | AAAAACCACAAAACCTGTGCTGT |
| 26 | insert of pBLR2540 | ATGAGATCAACAGCACAGGTTTT |
| 27 | insert of pBLR2541 | ATATTTATTTTCATTTTAGTCTG |
| 28 | insert of pBLR2566 | ATTTTCATTTTAGTCTGTCTTCT |
| 29 | insert of pBLR2567 | TCATTTTAGTCTGTCTTCTTGGT |
| 30 | insert of pBLR2568 | TAGTCTGTCTTCTTGGTTGCTGT |
| 31 | insert of pBLR2570 | GATATTATCTAAGTTTGAATATA |
| 32 | insert of pBLR2571 | TCTAAGTTTGAATATAAGGCTAT |
| 33 | insert of pBLR2572 | ATAGCCTTATATTCAAACTTAGA |
| 34 | insert of pBLR2576 | AATAATTTTTAAAATAGTATTCT |
| 35 | insert of pBLR2573 | AATATTTATAGCCTTATATTCAA |
| 36 | insert of pBLR2574 | TTAAATATTTATAGCCTTATATT |
| 37 | insert of pBLR2577 | TTAAAATAGTATTCTTGGTAATT |
| 38 | insert of pBLR2575 | TAAAAATTATTAAATATTTATAG |
| 39 | insert of pBLR2527 | TTGGTAATTGAATTATTCTTCTG |
| 40 | insert of pBLR2526 | CCAAGAATACTATTTTAAAAATT |
| 41 | insert of pBLR2579 | AATTATTCTTCTGTTTAAAGGCA |
| 42 | insert of pBLR2578 | AATTACCAAGAATACTATTTTAA |
| 43 | insert of pBLR2522 | TTCTTCTGTTTAAAGGCAGAAGA |
| 44 | insert of pBLR2529 | TTCTGTTTAAAGGCAGAAGAAAT |
| 45 | insert of pBLR2536 | CTTCTGCCTTTAAACAGAAGAAT |
| 46 | insert of pBLR2523 | TTTCTTCTGCCTTTAAACAGAAG |
| 47 | insert of pBLR2580 | AACATCATCCTGAGTTTTTCTGT |
| 48 | insert of pBLR2530 | CTACAGAAAAACTCAGGATGATG |
| 49 | insert of pBLR2581 | GGCTCTGATTCCTACAGAAAAAC |
| 50 | insert of pBLR2533 | TGAAACAAATGCATAATCTAAGT |
| 51 | insert of pBLR2532 | GTTTCAAAATATTGGGCTCTGAT |
| 52 | insert of pBLR2524 | TGCATTTGTTTCAAAATATTGGG |
| 53 | insert of pBLR2535 | CTTTCCATTTGACTTAGATTATG |
| 54 | insert of pBLR2521 | TTACTTCTTGTTTTCTTCAGTAT |
| 55 | insert of pBLR2525 | CTTCTTGTTTTCTTCAGTATTTA |
| 56 | insert of pBLR2531 | AACAATCCTTTTTTTTCTTCCCT |
| 57 | insert of pBLR2582 | TTAAATACTGAAGAAAACAAGAA |
| 58 | insert of pBLR2558 | AAACATCCTAGGTAAAAAAAAAA |
| 59 | insert of pBLR2556 | TATTAATAAGATAACCTTGTAAG |
| 60 | insert of pBLR2569 | AAACTTAGATAATATCTAATACT |
| 61 | insert of pBLR2534 | GACTTAGATTATGCATTTGTTTC |
| 62 | gRNA sequence for B-GEn.1 human albumin target 1 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CTTCTATTGTTCAACTTTTATTCT |
| 63 | gRNA sequence for B-GEn.1 human albumin target 2 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CTTCAACTTTTATTCTATTTTCCC |
| 64 | gRNA sequence for B-GEn.1 human albumin target 3 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CTATTTTCCCAGTAAAATAAAGTT |
| 65 | gRNA sequence for B-GEn.1 human albumin target 4 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CTCCCAGTAAAATAAAGTTTTAGT |
| 66 | gRNA sequence for B-GEn.1 human albumin target 5 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CTACTGGGAAAATAGAATAAAAGT |
| 67 | gRNA sequence for B-GEn.1 human albumin target 6 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CTTTAAAGATGCAGAGTTTACTAA |
| 68 | gRNA sequence for B-GEn.1 human albumin target 7 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CTTTTGGCATTTATTTCTAAAATG |
| 69 | gRNA sequence for B-GEn.1 human albumin target 8 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CTGGCATTTATTTCTAAAATGGCA |
| 70 | gRNA sequence for B-GEn.1 human albumin target 9 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CATTTCTAAAATGGCATAGTATTT |
| 71 | gRNA sequence for B-GEn.1 human albumin target 10 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CCTAAAATGGCATAGTATTTTGTA |
| 72 | gRNA sequence for B-GEn.1 human albumin target 11 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CTAGAAATAAATGCCAAAATAATT |
| 73 | gRNA sequence for B-GEn.1 human albumin target 12 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CTGTATTTGTGAAGTCTTACAAGG |
| 74 | gRNA sequence for B-GEn.1 human albumin target 13 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CGTGAAGTCTTACAAGGTTATCTT |
| 75 | gRNA sequence for B-GEn.1 human albumin target 14 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CATAAAATTCAAACATCCTAGGTA |
| 76 | gRNA sequence for B-GEn.1 human albumin target 15 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CATAAGATAACCTTGTAAGACTTC |
| 77 | gRNA sequence for B-GEn.1 human albumin target 16 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CTGACCTTTTTTTTTTTTTACCTA |
| 78 | gRNA sequence for B-GEn.1 human albumin target 17 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CTTTAGTGACTGTAATTTTCTTTT |
| 79 | gRNA sequence for B-GEn.1 human albumin target 18 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CCAGTCACTAAACAATTCTGACCT |
| 80 | gRNA sequence for B-GEn.1 human albumin target 19 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CTCTTTTGCGCACTAAGGAAAGTG |
| 81 | gRNA sequence for B-GEn.1 human albumin target 20 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CTGTGAAGTTTCAGTCACTCTAAG |
| 82 | gRNA sequence for B-GEn.1 human albumin target 21 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CAATTCATAACTATCCCAAAGACC |
| 83 | gRNA sequence for B-GEn.1 human albumin target 22 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CAATCTTCAACCCTATTCTGTGAA |
| 84 | gRNA sequence for B-GEn.1 human albumin target 23 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CATAACTATCCCAAAGACCTATCC |
| 85 | gRNA sequence for B-GEn.1 human albumin target 24 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CCACTATGCTTTATTTAAAAACCA |
| 86 | gRNA sequence for B-GEn.1 human albumin target 25 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CAAAAACCACAAAACCTGTGCTGT |
| 87 | gRNA sequence for B-GEn.1 human albumin target 26 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CATGAGATCAACAGCACAGGTTTT |
| 88 | gRNA sequence for B-GEn.1 human albumin target 27 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CATATTTATTTTCATTTTAGTCTG |
| 89 | gRNA sequence for B-GEn.1 human albumin target 28 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CATTTTCATTTTAGTCTGTCTTCT |
| 90 | gRNA sequence for B-GEn.1 human albumin target 29 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CTCATTTTAGTCTGTCTTCTTGGT |
| 91 | gRNA sequence for B-GEn.1 human albumin target 30 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CTAGTCTGTCTTCTTGGTTGCTGT |
| 92 | gRNA sequence for B-GEn.1 human albumin target 31 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CGATATTATCTAAGTTTGAATATA |
| 93 | gRNA sequence for B-GEn.1 human albumin target 32 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CTCTAAGTTTGAATATAAGGCTAT |
| 94 | gRNA sequence for B-GEn.1 human albumin target 33 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CATAGCCTTATATTCAAACTTAGA |
| 95 | gRNA sequence for B-GEn.1 human albumin target 34 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CAATAATTTTTAAAATAGTATTCT |
| 96 | gRNA sequence for B-GEn.1 human albumin target 35 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CAATATTTATAGCCTTATATTCAA |
| 97 | gRNA sequence for B-GEn.1 human albumin target 36 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CTTAAATATTTATAGCCTTATATT |
| 98 | gRNA sequence for B-GEn.1 human albumin target 37 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CTTAAAATAGTATTCTTGGTAATT |
| 99 | gRNA sequence for B-GEn.1 human albumin target 38 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CTAAAAATTATTAAATATTTATAG |
| 100 | gRNA sequence for B-GEn.1 human albumin target 39 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CTTGGTAATTGAATTATTCTTCTG |
| 101 | gRNA sequence for B-GEn.1 human albumin target 40 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CCCAAGAATACTATTTTAAAAATT |
| 102 | gRNA sequence for B-GEn.1 human albumin target 41 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CAATTATTCTTCTGTTTAAAGGCA |
| 103 | gRNA sequence for B-GEn.1 human albumin target 42 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CAATTACCAAGAATACTATTTTAA |
| 104 | gRNA sequence for B-GEn.1 human albumin target 43 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CTTCTTCTGTTTAAAGGCAGAAGA |
| 105 | gRNA sequence for B-GEn.1 human albumin target 44 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CTTCTGTTTAAAGGCAGAAGAAAT |
| 106 | gRNA sequence for B-GEn.1 human albumin target 45 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CCTTCTGCCTTTAAACAGAAGAAT |
| 107 | gRNA sequence for B-GEn.1 human albumin target 46 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CTTTCTTCTGCCTTTAAACAGAAG |
| 108 | gRNA sequence for B-GEn.1 human albumin target 47 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CAACATCATCCTGAGTTTTTCTGT |
| 109 | gRNA sequence for B-GEn.1 human albumin target 48 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CCTACAGAAAAACTCAGGATGATG |
| 110 | gRNA sequence for B-GEn.1 human albumin target 49 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CGGCTCTGATTCCTACAGAAAAAC |
| 111 | gRNA sequence for B-GEn.1 human albumin target 50 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CTGAAACAAATGCATAATCTAAGT |
| 112 | gRNA sequence for B-GEn.1 human albumin target 51 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CGTTTCAAAATATTGGGCTCTGAT |
| 113 | gRNA sequence for B-GEn.1 human albumin target 52 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CTGCATTTGTTTCAAAATATTGGG |
| 114 | gRNA sequence for B-GEn.1 human albumin target 53 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CCTTTCCATTTGACTTAGATTATG |
| 115 | gRNA sequence for B-GEn.1 human albumin target 54 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CTTACTTCTTGTTTTCTTCAGTAT |
| 116 | gRNA sequence for B-GEn.1 human albumin target 55 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CCTTCTTGTTTTCTTCAGTATTTA |
| 117 | gRNA sequence for B-GEn.1 human albumin target 56 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CAACAATCCTTTTTTTTCTTCCCT |
| 118 | gRNA sequence for B-GEn.1 human albumin target 57 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CTTAAATACTGAAGAAAACAAGAA |
| 119 | gRNA sequence for B-GEn.1 human albumin target 58 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CAAACATCCTAGGTAAAAAAAAAA |
| 120 | gRNA sequence for B-GEn.1 human albumin target 59 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CTATTAATAAGATAACCTTGTAAG |
| 121 | gRNA sequence for B-GEn.1 human albumin target 60 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CAAACTTAGATAATATCTAATACT |
| 122 | gRNA sequence for B-GEn.1 human albumin target 61 | CTGGATATAATAAAGATGTTTAGCTATAGGCTAATA AGATAGTTGTGTCAAGTGCTTCGGAGACCTAACACG TCATCCAGTCACAACGGCTATCTATATTTCCACTAA CGACTTAGATTATGCATTTGTTTC |
| 123 | pBLR2542 | |
| 124 | pBLR2543 | |
| 125 | pBLR2545 | |
| 126 | pBLR2546 | |
| 127 | pBLR2544 | |
| 128 | pBLR2547 | |
| 129 | pBLR2549 | |
| 130 | pBLR2550 | |
| 131 | pBLR2551 | |
| 132 | pBLR2552 | |
| 133 | pBLR2548 | |
| 134 | pBLR2553 | |
| 135 | pBLR2554 | |
| 136 | pBLR2557 | |
| 137 | pBLR2555 | |
| 138 | pBLR2559 | |
| 139 | pBLR2561 | |
| 140 | pBLR2560 | |
| 141 | pBLR2562 | |
| 142 | pBLR2563 | |
| 143 | pBLR2565 | |
| 144 | pBLR2564 | |
| 145 | pBLR2537 | |
| 146 | pBLR2538 | |
| 147 | pBLR2539 | |
| 148 | pBLR2540 | |
| 149 | pBLR2541 | |
| 150 | pBLR2566 | |
| 151 | pBLR2567 | |
| 152 | pBLR2568 | |
| 153 | pBLR2570 | |
| 154 | pBLR2571 | |
| 155 | pBLR2572 | |
| 156 | pBLR2576 | |
| 157 | pBLR2573 | |
| 158 | pBLR2574 | |
| 159 | pBLR2577 | |
| 160 | pBLR2575 | |
| 161 | pBLR2527 | |
| 162 | pBLR2526 | |
| 163 | pBLR2579 | |
| 164 | pBLR2578 | |
| 165 | pBLR2522 | |
| 166 | pBLR2529 | |
| 167 | pBLR2536 | |
| 168 | pBLR2523 | |
| 169 | pBLR2580 | |
| 170 | pBLR2530 | |
| 171 | pBLR2581 | |
| 172 | pBLR2533 | |
| 173 | pBLR2532 | |
| 174 | pBLR2524 | |
| 175 | pBLR2535 | |
| 176 | pBLR2521 | |
| 177 | pBLR2525 | |
| 178 | pBLR2531 | |
| 179 | pBLR2582 | |
| 180 | pBLR2558 | |
| 181 | pBLR2556 | |
| 182 | pBLR2569 | |
| 183 | pBLR2534 | |
| 184 | pBLR709 | |
| 185 | SEQ ID NO:2 from WO2022/258753 | |
| 186 | SEQ ID NO:39 from WO2022/258753 | |

### 4. Examples

### 4.1. Example 1- Screening Methods to detect nuclease activity on the albumin intron 1 locus in mammalian cells

### Cloning and plasmid preparation

The cloning of the B-Gen.1 expression vector pBLR709, the transfection in HEK293T cells, AMP-Seq, DNA library preparation, and NGS analysis is described in detail in WO2022258753 (with the nuclease of SEQ ID NO: 2).

The cloning of the human albumin guide RNA expression vectors was done as follows. Acceptor vector pBLR1936 was digested with Bbsl-HF (New England Biolabs) according to manufacturers guidelines. Sense and antisense strand matching oligonucleotides, which encompass the respective spacer sequence (see document AII_Sequences_ALB_guides_TSC_template_as_sent.xlsx) of SEQID 1-61 were annealed to generate small double stranded DNA fragments with fitting overhangs. Annealed oligos were incubated with pre-cut pBLR1936 in a Golden Gate reaction according to manufacturer's guidelines to generate pBLR2521-2582. Single Golden Gate reactions were transformed and propagated in Top10 electrocompetent *E.Coli.* Plasmids were Sanger sequenced and positive clones were propagated as medium (midi) cultures and isolated with the NuceloSnap Plasmid Midi Kit for plasmid DNA (Macherey Nagel).

### Cell culture

HEK293T (ATCC^{®} CRL-3216^{™}) cells were cultivated in DMEM medium with 10% FCS and 1% Pen/Strep in T75 flasks. Cells were passaged every third day.

### Transfection in HEK293T cells

One day prior transfection cells were split as described above and counted with the Luna-FL^{™} Dual Fluorescence Cell Counter Counter (Biocat) according to manufacturer's guidelines. Cells were plated in a poly-D-lysine coated 96 well plate at a density of 18,000 per well in 100µl Medium.

On the day of transfection medium was changed. LipoD293^{™} (SignaGen) reagent was adjusted to room temperature shortly before transfection (~10min). At the day of transfection plasmid 140ng of pBLR709 DNA was mixed with the 60ng of guide RNA plasmid or no guide RNA plasmid (nuclease only control) DNA and medium without antibiotics and FCS was added to 10µl total. In another tube 10µl DMEM without additives was mixed with 0.3µl of LipoD293. The Lipo mixture was added to the DNA mixture. Plate was sealed, shaked, spun down at 300g for 10 seconds and put at room temperature for 15 minutes. After that transfection mix was added to the cells.

After three days post transfection cells were harvested. Medium was aspirated and 50µl of PBS was added per well. After that 25µl of TrypLE was added on top. Cells were trypsinized for 15 minutes at 37C. 60 µ! of PBS was added for resuspension. 60 µl of the cell suspension was added to PCR tubes in a 96 well plate format. Cells were spun down at 300g for 3 minutes. Supernatant was discarded and cells were lysed in 60µl Lysis buffer (Liu lysis buffer composition) + 1:800 Proteinase K (company) in a heat cycler (insert protocol)

### AMP-Seq

To generate the amplicon endpoint PCR with barcoded primer was performed. The PCR reaction was prepared as follows:
12,5 µL 2x Q5 Mastermix, 8 µL H2O, 3µL Barcoded primer, 1.5 µL crude lysis genomic DNA
Following cycler conditions were used: Step 1: 98C for 30sec, Step 2: 98C for 10sec, Step 3: 66C for 20sec, Step 4: 72C for 20sec Step 5: 32 cycles of steps 2-4, Step 6: 72C for 2 min, Step 7: 12C hold For each guide RNA amplicon number 18, 27, 42, 50, 51 and 57 were designated (see Table 3).

**Table 3 Amplicon designations for each guide RNA**

| ***SEQ ID No.*** | ***pBLR*** | ***amplicon*** | ***Activity in* %** |
|---|---|---|---|
| 1 | pBLR2542 | 51 | 2,84 |
| 2 | pBLR2543 | 51 | 0,94 |
| 3 | pBLR2545 | 51 | 13,60 |
| 4 | pBLR2546 | 51 | 74,47 |
| 5 | pBLR2544 | 51 | 64,69 |
| 6 | pBLR2547 | 51 | 56,38 |
| 7 | pBLR2549 | 18, 51 | -0,21 |
| 8 | pBLR2550 | 18, 51 | 1,90 |
| 9 | pBLR2551 | 18, 51 | 55,48 |
| 10 | pBLR2552 | 18, 51 | n/a |
| 11 | pBLR2548 | 18, 51 | 18,42 |
| 12 | pBLR2553 | 18, 51 | 26,38 |
| 13 | pBLR2554 | 18, 51 | 42,06 |
| 14 | pBLR2557 | 18, 51 | 47,03 |
| 15 | pBLR2555 | 18, 51 | 50,77 |
| 16 | pBLR2559 | 18, 51 | 16,66 |
| 17 | pBLR2561 | 18, 51 | 5,49 |
| 18 | pBLR2560 | 18, 51 | 1,58 |
| 19 | pBLR2562 | 18, 51, 42 | 2,64 |
| 20 | pBLR2563 | 18,42 | 56,61 |
| 21 | pBLR2565 | 18,42 | 16,76 |
| 22 | pBLR2564 | 18,42 | 19,13 |
| 23 | pBLR2537 | 18,42 | 0,25 |
| 24 | pBLR2538 | 42 | 0,32 |
| 25 | pBLR2539 | 42 | 18,11 |
| 26 | pBLR2540 | 42 | 45,69 |
| 27 | pBLR2541 | 42 | -0,04 |
| 28 | pBLR2566 | 42 | -0,39 |
| 29 | pBLR2567 | 42 | -0,28 |
| 30 | pBLR2568 | 42 | -0,02 |
| 31 | pBLR2570 | 27 | 4,72 |
| 32 | pBLR2571 | 27 | 26,07 |
| 33 | pBLR2572 | 27 | 7,98 |
| 34 | pBLR2576 | 27 | n/a |
| 35 | pBLR2573 | 27 | -0,32 |
| 36 | pBLR2574 | 27 | n/a |
| 37 | pBLR2577 | 27 | n/a |
| 38 | pBLR2575 | 27 | n/a |
| 39 | pBLR2527 | 27 | 43,86 |
| 40 | pBLR2526 | 27 | 0,93 |
| 41 | pBLR2579 | 27 | 3,78 |
| 42 | pBLR2578 | 27 | 71,34 |
| 43 | pBLR2522 | 27 | 6,17 |
| 44 | pBLR2529 | 27 | 16,45 |
| 45 | pBLR2536 | 27 | 6,44 |
| 46 | pBLR2523 | 27 | 14,59 |
| 47 | pBLR2580 | 27 | 1,72 |
| 48 | pBLR2530 | 27 | 0,35 |
| 49 | pBLR2581 | 27 | 9,92 |
| 50 | pBLR2533 | 27 | n/a |
| 51 | pBLR2532 | 27 | 15,57 |
| 52 | pBLR2524 | 27 | 54,35 |
| 53 | pBLR2535 | 57 | n/a |
| 54 | pBLR2521 | 57 | 47,83 |
| 55 | pBLR2525 | 57 | 48,30 |
| 56 | pBLR2531 | 57 | 12,62 |
| 57 | pBLR2582 | 57 | n/a |
| 58 | pBLR2558 | 51,18 | 46,13 |
| 59 | pBLR2556 | 51,18 | 66,67 |
| 60 | pBLR2569 | 27 | 18,42 |
| 61 | pBLR2534 | 27 | n/a |

### DNA library preparation and NGS analysis

All PCR reactions were pooled. For that 5 µl of single reactions were pooled. Total volume of pooled PCR was mixed with equal amount of AMPure XP Bead cleanup. After incubation on a rotor for 5 minutes mixture was placed on a magnet and DNA was extracted according to manufacturer's guidelines. To ligate Illumina adapters to the sheared fragments ends were repaired. For that the NEBNext^{®} Ultra^{™} II End Repair/dA-Tailing Module was used. In total 2µg of the amplicon (up to 80 µL) was incubated with 6µl of NEBNext Ultra II End Prep Enzyme Mix and 14 µ! NEBNext Ultra II End Prep Reaction Buffer. After that it was filled with H2O ad 100µl. The reaction in Thermocycler used the following protocol: 30 minutes @ 20°C, 30 minutes @ 65°C, Hold at 4°C. Samples were cleaned up with AMPure XP Bead Cleanup and eluted in 32 µl EB buffer.For the ligation we use the Blunt/TA Ligase Maser Mix from NEB and Illumina adapters from the TruSeq DNA PCR-Free LT Sample Prep Kit. 30 µL of End-prepped DNA was mixed with 2µL Illumina and 32 µL of Blunt/TA Ligase Master Mix. This was incubated at 1h at 21°C in a thermocycler. Then 34 µl H2O was added to a total of 100µl and AMPure XP beads cleanup was performed with a final elution step in 50µl EB buffer. Double stranded DNA library was measured with the Qubit 4 HS system according to manufacturer's guidelines. DNA library was diluted to 1 nm with RSB buffer and then dilute to 450 pM. DNA library was loaded onto a NextSeq 1000 Sequencer (Illumina) using the manufacturers protocol.

Raw fastq files were quality trimmed using cutadapt version 1.18 (https://cutadapt.readthedocs.io/en/v1.18/index.html) with a minimum quality score of Q30. Filtered reads were joined using fastq-join version 1.3.1 and demultiplexed using a custom demultiplexing as described in WO2022258753 (storage.googleapis.com). the demultiplexed files were subsequently analyszed using the CRISPresso v. 1.0.13 (doi: 10.1038/nbt.3583). Final values were plotted with GraphPad Prism 9.50 (GraphPad Software).

## Claims

1. A system comprising:
(a) a deoxyribonucleic acid (DNA) endonuclease or nucleic acid encoding said DNA endonuclease, selected from SEQ ID NO: 185 or SEQ ID NO: 186, or any sequence thereof which is 95%, preferably, 98% identical, more preferably 99% identical to these sequences; and
(b) a guide RNA (gRNA) from any one of SEQ ID NOs: 62 to 122, or nucleic acid encoding the gRNA, or any or any sequence thereof which is 95%, preferably, 98%, more preferably 99% identical to these sequences; and
(c) a donor template comprising a nucleic acid sequence encoding a gene-of-interest (GOI) or functional derivative thereof.

2. A system according to claim 1, in which the guide RNA is selected from SEQ ID NOs:

62,63,64,65,66,67,68,69,70,72,73,74,75,76,77,78,79,80,81,82,83,84,85,86,87,88,
89,90,91,92,93,94,96,100,101,102,103,104,105,106,107,108,109,110,112,113,11
5,116,117,119,120,121.

3. A system according to claim 1, in which the guide RNA is selected from SEQ ID NOs:

64,65,66,67,70,72,73,74,75,76,77,81,82,83,86,87,93,100,103,105,107,112,113,11
5,116,117,119,120,121.

4. A system according to claim 1, in which the guide RNA is selected from SEQ ID NOs: 65,66,67,70,73,74,75,76,81,87,93,100,103,113,115,116,119,120.

5. A system according to claim 1, in which the guide RNA is selected from SEQ ID NOs: 65,66,67,70,76,81,103,113,120.

6. A system according to claim 1, in which the guide RNA is selected from SEQ ID NOs: 65,103.

7. A method of editing a genome in a cell, the method comprising:
providing the following to the cell:
(a) a deoxyribonucleic acid (DNA) endonuclease or nucleic acid encoding said DNA endonuclease, selected from SEQ ID NO: 185 or SEQ ID NO: 186, or any sequence thereof which is 95%, preferably, 98% identical, more preferably 99% identical to these sequences; and
(b) a guide RNA (gRNA) from any one of SEQ ID NOs: 62 to 122, or nucleic acid encoding the gRNA, or any or any sequence thereof which is 95%, preferably, 98%, more preferably 99% identical to these sequences; and
(c) a donor template comprising a nucleic acid sequence encoding a gene-of-interest (GOI) or functional derivative thereof,
wherein the method is not a method for treatment of the human or animal body by therapy.

8. The system of any of claims 1 to 6 for use in the treatment of a disorder or health condition in a subject, wherein (a), (b) and (c) are to be provided to a cell in the subject.

9. The system of any one of claims 1 to 6, or the method of claim 7, wherein the components (a) to (c) are encoded by one or separate viral vectors, optionally, such viral vector is an AAV.

10. The system of any one of claims 1 to 6, or the method of claim 7, wherein the components (a) to (c) are formulated in one or separate liposome or lipid nanoparticle(s).
